# EUROPEAN PATENT APPLICATION

(11) **EP 4 389 912 A1**
(43) Date of publication of application: **26.06.2024**
(21) Application number: 22216302.4
(22) Date of filing: 23.12.2022
(51) Int. Cl.: C12Q 1/6813

(54) **METHOD FOR IMAGING, MICROSCOPY SYSTEM, SET OF STRANDS**

(71) Applicant: Ludwig-Maximilians-Universität München, 80539 München (DE)
(72) Inventor: Dr.Tinnefeld, Philip, Munich (DE); Zähringer, Jonas, Munich (DE); Cole, Fiona, Munich (DE); Bohlen, Johann, Munich (DE)
(74) Representative: Vossius & Partner Patentanwälte Rechtsanwälte mbB

(57) **Abstract**

The present invention relates to a method for imaging a target structure (12), including applying a plurality of docking strands (14) to the target structure (12) such that the docking strands (14) dock to the target structure (12) and applying one or more scanning strands (16) to the target structure (12). Each scanning strand (16) has a first docking section (18), which docks to the target structure (12), and at least a second docking section (20), which simultaneously docks to at least one of the docking strands (14). Each scanning strand (16) includes a marking (22) which is at least temporarily attached to the respective scanning strand (16) and is detectable by a detection means (24) to detect a location of the marking (22) along the target structure (12).

The present invention further relates to a microscopy system (10) and a set (26) of strands for use with a microscope for imaging at least one target structure (12).

## Description

Technologies for imaging a target structure can be used in a number of different fields and applications. In this respect, super-resolution microscopy is a growing field with a variety of applications and/or purposes, e.g., in biology and/or nanoscience.

Super-resolution fluorescence microscopy may include successive single-molecule localizations of a target structure to individually determine a position of a plurality of molecules of interest, respectively, within a field of view. In particular, super-resolution fluorescence microscopy, which may provide imaging at a sub-diffraction limit, theoretically unlimited, spatial resolution, can use transient binding of fluorescent or fluorogenic ligands. One form of super-resolution fluorescence microscopy is known as DNA-PAINT ("DNA Points Accumulation for Imaging in Nanoscale Topography").

Super-resolution fluorescence microscopy, such as DNA-PAINT, may allow complex and dynamic processes within biological systems to be examined and studied in relatively great detail. However, despite the benefits, several drawbacks still exist in the super-resolution fluorescence microscopy known from the prior art, such as DNA-PAINT.

For instance, on the one hand it is generally required and/or desired to keep the concentrations of ligands in solution relatively low since greater concentrations may reduce a Signal to Background Ratio (SBR) which may reduce the precision of the measurement(s)/localizations which is generally undesired. Moreover, greater concentrations of ligands may cause double-binding events, i.e., in which a plurality of ligands bind simultaneously, which could impede an accurate determination of localizations, e.g., molecule localizations.

However, on the other hand, lower concentrations of ligands may increase the measurement/localization times in super-resolution fluorescence microscopy which may reduce the efficiency and/or increase the costs of the imaging procedure.

Moreover, at reasonable concentrations the stochastic binding process of the ligands to the target structure in super-resolution fluorescence microscopy known from the prior art occurs with a relatively low kinetic rate which leads to comparatively long measurement times which may hamper 3D measurements, in particular in measurements in which sample drift may be a problem.

It is therefore an object of the present invention to provide an improved means for super-resolution imaging.

This object is achieved by a method for imaging at least one target structure, as defined by the features of claim 1. Preferred embodiments are defined by the features of the dependent claims, respectively.

The method may include applying a plurality of docking strands to the target structure such that the docking strands dock to the target structure.

The method may further include applying one or more scanning strands to the target structure. Each scanning strand may have a first docking section, which docks to the target structure, and at least a second docking section, which docks to at least one of the plurality of docking strands, at least partially simultaneously,

Each scanning strand may include at least one marking which is at least partially and at least temporarily attached to the respective scanning strand. The marking may be detectable by at least one detection means to detect a location of the marking along the target structure, at least when the respective scanning strand is docked to a respective docking strand. A plurality of markings, which may be interconnected by an intermediate medium, e.g., a strand, e.g., a DNA strand or an RNA strand, may be attached to one or more scanning strands.

In other words, once at least some of the docking strands have docked to the target structure, at the first docking section, the docking strands may provide a plurality of docking targets for the one or more scanning strands, in particular the second docking section, to dock to, once the one or more scanning strands have been applied to the target structure.

Configuring the scanning strands to have different docking sections, i.e., a first docking section and at least a second docking section, respectively, may allow the respective scanning strand to at least partially simultaneously dock to at least one of the docking strands and to the target structure. This may allow the respective scanning strand to dock to the respective target structure at the first docking section, and remain docked to the same location on the target structure, for a certain time period, while the second docking section of the scanning strand docks to one or more docking strands successively, thus effectively "scanning" an area about the location of the target structure at which the first docking section is docked. This may provide a relatively large number of localizations in a relatively small area of the target structure. This may also allow molecular clusters to be imaged relatively quickly and in a bunched manner which may reduce the influence of drift.

Moreover, this may enable a concentration of markings, e.g., ligands, to be reduced which may reduce the likelihood of double-binding events, while increasing the imaging speed over the technologies known from the prior art. As discussed above, lower concentrations of markings/marked strands and imaging speed are counteractive in the technology known from the prior art. Thus, there is a tradeoff between the concentrations of markings/marked strands and the imaging speed. The present disclosure effectively breaks this counteractive relationship between the concentrations of markings/marked strands and the imaging speed via the configuration described above. In particular, the first binding event of the scanning strand(s) to the target structure may be considered to generate a relatively high local concentration of the marker, effecting the subsequent binding/scanning events.

The scanning strand(s) may transiently, or alternatively permanently, dock to the target structure, at the first docking section of the respective scanning strand, and/or the scanning strand(s) may transiently dock to the respective docking strand, at the second docking section of the scanning strand. Preferably, the respective scanning strand may transiently, alternatively permanently, dock to an x number of different locations of the target structure successively per time unit, at the first docking section of the respective scanning strand, and the scanning strand may transiently dock to a y number of different docking strands successively per time unit, at the second docking section of the respective scanning strand, wherein y is greater than x. The scanning strand(s) may be configured to undock from the target structure and the docking strands, respectively, without user intervention. In other words, the scanning strand(s) may be configured to undock from the target structure and the docking strands passively, e.g., after a certain time period or time threshold.

The marking may be detectable by the detection means by detecting an intensity and/or a scattering of the marking, e.g., of a signal, e.g., a fluorescent signal, which is emitted by the marking, at least when the respective scanning strand is docked to a respective docking strand. For instance, the marking may be optically detectable by the detection means. However, a variety of further detection mechanism may be employed. For instance, an energy transfer between the marking and a further structure, e.g., a metal- or graphene-layer, may be detected to detect a location of the marking along the target structure.

One or more of the markings may be replaced by one or more replacement markings. For instance, the scanning strand(s) may be replaced by one or more replacement scanning strands, which preferably have markings pre-attached thereto. In particular if the markings are configured as a dye, a plurality of localizations at different binding/docking sites may cause a photon budget of the dye to be depleted, which may cause photobleaching. Hence, replacing at least some of the markings may provide a countermeasure to prevent, or at least reduce the risk of, photobleaching. Alternatively, one or more of the markings may be replaced while maintaining the scanning strand(s) on the target structure. For instance, the marking(s) may be periodically removed from the scanning strand(s), e.g., by replacing a carrier, e.g., a separate strand, which attaches/docks to the scanning strand(s) and which carries the marking(s) by a replacement carrier. Moreover, providing one or more replacement markings strands may effectively increase an amount or number of available markings, and thus of potential detectable docking/binding events, e.g., within a diffraction limited volume.

A duration during which the markings are attached to the respective scanning strand(s) may be varied and/or adapted. For instance, a duration during which the markings are attached to the respective scanning strand(s) may be shorter than a duration during which the respective scanning strand is docked to a certain location on the target structure. For instance, the scanning strand(s), or at least some of the scanning strands, may be permanently, at least during the imaging process, docked to a location on the target structure, while one or more markings periodically dock and undock to and from the scanning strand(s). This may allow the scanning strand(s) to be periodically activated by the one or more markings and thus may allow multiple scanning strands to be docked to the target structure within a diffraction limited area at least partially simultaneously.

The claimed method is not limited to a certain order of steps, e.g., the order in which the steps are described above. The scanning strand(s) and the docking strands may be applied at least partially simultaneously to the target structure. Preferably, the docking strands may at least partially be applied to the target structure before the scanning strand(s) is/are applied to the target structure. The scanning strand(s) and/or the docking strands may be diffused, preferably freely diffused, in a solution, respectively, which is applied to the target structure.

For the scanning strand, the bond at the first docking section, i.e., between the first docking section and the target structure, may be configured to be stronger than a bond at the second docking section, i.e., between the second docking section and the respective docking strand. This may allow the bond at the first docking section to be maintained for a longer time period than the bond at the second docking section. This may allow the first docking section of the respective scanning strand to dock to the target structure, while the second docking section of the same scanning strand docks and undocks to and from a plurality of binding strands successively.

The term "least partially simultaneously" means that the docking event, in which a respective scanning strand docks to the target structure at the first docking section, and the docking event, in which the same scanning strand docks to at least one of the docking strands at the second docking section, may at least partially temporally overlap.

All docking strands may be at least partially/section-wise identical. For instance, all docking strands may be configured as DNA strands or RNA strands which have the same DNA/RNA sequence at least in one or more sections thereof. For instance, a docking sequence of the scanning strand(s), e.g., at the second docking section, may have a length which is greater than a length of a corresponding docking sequence in at least a section of the docking strand(s), which cooperates with the docking sequence of the scanning strand(s), e.g., at the second docking section. However, the docking sequence in at least a section of the docking strand(s) may at least partially overlap, i.e., may be at least partially identical, with at least a section of the docking sequence of the scanning strand(s).

Furthermore, the sequence of the second docking section of the scanning strand(s) and the sequence at a section of the docking strands, which docks to the second docking section, may be completely identical, e.g., including in length.

The first docking section may be configured to dock directly to the target structure. Alternatively, the first docking section may be configured to dock indirectly, e.g., via an intermediate medium, e.g., an intermediate strand, to the target structure. For instance, the first docking section may be configured to dock to at least one docking strand, as an intermediate structure between the first docking section and the target structure. Thus, in this case, both the first docking section and the second docking section may dock to respective docking strands at least partially simultaneously.

Each scanning strand may individually dock to at least a section of the target structure, directly or indirectly. The first docking section and the second docking section(s) may be any section of the scanning strand, e.g., an end section and/or an intermediate section. Preferably, the first docking section of the scanning strand is an end section of the scanning strand. Preferably, the second docking section is an end section of the scanning strand, preferably an end section which is opposite from the first docking section, and the marking is attached/attachable to the second docking section. This may allow the marking to be arranged relatively close to the location of the target structure at which the docking strand is docked which may increase the accuracy of the imaging of the target structure. Alternatively, the second docking section may not be an end section of the scanning strand. Instead, the second docking section may be arranged between the first docking section and an end section of the scanning strand.

The marking may be a label, preferably a fluorescent label, preferably a label which is configured to only emit a fluorescent signal when the respective scanning strand is docked to the respective docking strand. The marking may be a dye, preferably a fluorescent dye. The marking may be a ligand. The marking(s) is/are preferably optically detectable by at least one imaging system and/or one or more optically sensors. The imaging system may include at least one optical system and at least one imaging sensor. The imaging system may be configured as a camera and/or an optical system of a microscope. Alternatively, further methods of determining the location are feasible, e.g., by determining coordinates, e.g., by generating a list of coordinates, localizations, etc. A plurality of ligands, which may be interconnected by an intermediate medium, e.g., a strand, e.g., a DNA strand or an RNA strand, may be attached to one or more scanning strands. A double ligand, e.g., two nanobodies, or a snap tag and a halo tag, may be employed and attached to one or more scanning strands. The double ligand may bond/dock to two different positions of the target structure, e.g., within a cell. A strand, e.g., a DNA strand or an RNA strand, which may connect the ligands may have a plurality of different sequences, e.g., to allow a precise colorization of the ligands.

The marking(s) may be attached permanently to the respective scanning strand The marking(s) may be attached to the respective scanning strand while the second docking section sequentially/successively docks and undocks to and from a plurality of docking strands.

The marking(s) may be attached directly to the respective scanning strand. Alternatively, the marking(s) may be attached indirectly to the respective scanning strand, e.g., via another strand.

The docking strands and/or the scanning strand(s) may be any type of strand, e.g., DNA strands having one or more DNA sequences and/or RNA strands having one or more RNA sequences.

The target structure may be any type of structure which is to be imaged. The target structure may include biological matter, preferably one or more cells, e.g., living cells, e.g., a single cell. For instance, the target structure may be a single or a plurality of strands, e.g., a DNA strand or an RNA strand, preferably an mRNA strand.

Preferably, the docking strands remain docked to substantially the same location on the target structure during the entire imaging process, or at least during a plurality of steps of the method, e.g., the above-identified steps of the method. However, the docking strands may periodically undock from the target structure, e.g., by undertaking one or more measures which cause one or more of the docking strands to undock from the target structure, e.g., to swap one or more docking strands, e.g., by replacing one more first docking strands by one of more second docking strands, which may be different from the one or more first docking strands.

Preferably, the method further includes applying one or more imaging strands to the target structure. The imaging strands may each include at least one marking, respectively, which is at least partially attached to the respective imaging strand. Preferably, at least one of the imaging strands docks to an imaging strand docking section of at least one of the scanning strands. Hence, the imaging strands may provide an intermediate structure, e.g., as a marking carrier, to attach the marking(s) to the respective scanning strand. For one, this may enable to more easily replace at least some of the markings, e.g., periodically, by new markings by replacing at least some of the imaging strands, which have already been applied to the target structure, with replacement imaging strands. In particular if the markings are configured as a dye, a plurality of localizations at different binding sites may cause the photon budget of the dye to be depleted, which may cause photobleaching, as discussed above. Hence, providing imaging strands which can be replaced/regenerated may provide a relatively reliable and simple countermeasure to prevent, or at least reduce the risk of, photobleaching. The imaging strands may be configured as DNA strands having one or more DNA sequences or RNA strands having one or more RNA sequences. Moreover, providing the one or more imaging strands may effectively increase an amount or number of available markings, and thus of potential detectable docking/binding events, e.g., within a diffraction limited volume

Preferably, a bonding strength at the imaging strand docking sections, i.e., between the imaging strands and the scanning strands, may be predetermined and/or adjusted and/or adapted, in particular relative to a bonding strength at the first docking section and the second docking section. This may provide a bonding hierarchy of at least two, preferably at least three, different bonding strengths and/or bonding durations, to be achieved.

Preferably, the imaging strand docking section is arranged at an end section of the scanning strands. Preferably, the end section is substantially opposite from the first docking section. This may allow the marking to be arranged relatively close to the location of the target structure at which the docking strand is docked which may increase the accuracy of the imaging of the target structure. Moreover, this may increase the effective usable length of the scanning strands for "scanning" an area about the location of the target structure at which the first docking section is docked, compared with a configuration in which the imaging strand docking section is not arranged at an end section of the scanning strands. This may enable the scanning strands to "scan", i.e., dock and undock to and from a plurality of docking strands successively, a relatively large area. In this case, the second docking section may not be an end section of the scanning strand. Instead, the second docking section may be arranged between the first docking section and the imaging strand docking section of the scanning strand.

Preferably, the second docking section is arranged between the imaging strand docking section and the first docking section. This may place the marking relatively closely to the target structure to increase the accuracy of the imaging. Moreover, this may maximize, or at least increase, a range of the scanning strands. Alternatively, the imaging strand docking section may be arranged between the first docking section and the second docking section.

Preferably, the method further includes:
capturing one or more images, preferably a plurality of images, preferably sequentially, of at least a section of the target structure with a super resolution by means of at least one imaging system, at least after the docking strands and the scanning strands have been applied to the target structure; and/or
generating one or more coordinates of at least some of the detected location(s) of the marking(s) along the target structure.

In fact, a wide variety of various means for recording the locations of the markings to image/identify the target structure may be employed. For instance, as detailed above, one or more images may be captured by means of at least one imaging system. However, recording the locations of the markings without relying on images e.g., by recording one or more coordinates of at least some of the detected location(s) of the marking(s) along the target structure, may require less resources and/or may be quicker than capturing one or more images.

Preferably, a stronger bond is generated at the first docking section than at the second docking section and/or a stronger bond is generated at the first docking section than at the imaging strand docking section and/or a stronger bond is generated at the imaging strand docking section than at the second docking section. This may provide a hierarchy of bonding strengths at the first docking section, the second docking section, and/or the imaging strand docking section. This may allow the docking/binding times of the first docking section, the second docking section, and/or the imaging strand docking section to be varied. The bonding strengths may be varied by adapting one or more properties at the respective bonding interface. For instance, in case the scanning strand(s) and the docking strands are configured as DNA strands or RNA strands, the first docking section may be configured with a DNA sequence or RNA sequence which is different than a DNA sequence or RNA sequence of the second docking section and/or the imaging strand docking section.

However, the first docking section, the second docking section and the imaging strand docking section can also have equal strength as upconcentrating is reached by the avidity effect.

Alternatively, the docking/binding at the first docking section, the second docking section and/or the imaging strand docking section may be at least partially based on various other interactions, besides via DNA/RNA interaction. For instance, the docking/binding at the first docking section, the second docking section and/or the imaging strand docking section may be based on one or more protein interactions. For instance, one or more proteins may be attached to the scanning strand(s), the docking strand(s) and/or the imaging strand(s) which provide, or at least contribute to, the docking/binding at the first docking section, the second docking section and/or the imaging strand docking section via interaction between the respective proteins. The bonding/docking strengths and/or bonding/docking durations at the first docking section, the second docking section and/or the imaging strand docking section may be varied by configuring and/or adapting an interaction between the proteins accordingly, e.g., by varying a strength and/or an intensity of the protein-to-protein interaction.

Preferably, a time period, during which the first docking section is docked to the target structure, is longer than a time period, during which the second docking section is docked to the docking strand. This may allow the respective scanning strand to dock to the respective target structure at the first docking section and remain docked to the same location on the target structure, while the second docking section of the scanning strand docks to one or more docking strands successively, thus effectively "scanning" an area about the location of the target structure at which the first docking section is docked.

Alternatively, or additionally, a time period, during which the first docking section is docked to the target structure, is longer than a time period, during which the imaging strand is docked to the imaging strand docking section. This may allow the marking to be replaced, by replacing the imaging strand by a replacement imaging strand, while the scanning strand remains docked to the target structure at the first docking section.

Alternatively, or additionally, a time period, during which the imaging strand is docked to the imaging strand docking section, is longer than a time period, during which the second docking section is docked to the docking strand. This may allow the same marking to be attached to the scanning strand, while the scanning strands docks to a plurality of docking strands successively.

Preferably, the at least one second docking section sequentially dislodges from the respective docking strand and bonds with one or more further docking strands.

Preferably, the scanning strand(s) is/are configured to periodically dislodge from a first location on the target structure and dock to a different location on the target structure. The scanning strand(s) may be dissolved into solution and/or the one or more scanning strand(s) may be replaced by one or more replacement scanning strands during the imaging process and/or between imaging processes.

Preferably, the second docking section sequentially docks to a plurality of docking strands, preferably at least two docking strands, preferably at least three docking strands, more preferably at least four docking strands, more preferably at least five docking strands, more preferably at least six docking strands, while the first docking section is docked to the target structure at substantially the same location on the target structure.

Preferably, the marking is a fluorescent element, preferably a fluorogen, preferably a fluorogenic ligand, which emits a fluorescent signal which is detectable by the detection means to detect a location of the marking along the target structure, at least, preferably only, when the respective scanning strand is docked to a respective docking strand. As an example, the marking may be an ATTO542 by ATTO-TEC GmbH.

Preferably, at least one marking is attached to an end section of the scanning strands, respectively, and/or at least one marking is attached to a section of the scanning strands which is arranged between the first docking section and the second docking section, respectively.

Preferably, the docking strands and/or the scanning strand(s) and/or the imaging strand(s) is/are configured as DNA strands having one or more DNA sequences or RNA strands having one or more RNA sequences.

Preferably, the first docking section has at least a first DNA sequence and the second docking section has at least a second DNA sequence which is different from the first DNA sequence. Configuring the first docking section and the second docking section with different DNA sequences may allow various bonding/docking strengths, and thus bonding/docking durations, i.e., durations of a bonded state, to be different/varied between the first docking section and the second docking section.

Preferably, the first docking section and the second docking section are connected to each other by at least one link section. The link section may include the imaging strand docking section. Alternatively, the first docking section and the imaging strand docking section may be connected to each other by the link section. For instance, the link section may include the second docking section.

Preferably, each scanning strand has a contour length of 5 nm to 700 nm, preferably 5 nm to 650 nm, more preferably 5 nm to 600 nm, more preferably 5 nm to 550 nm, more preferably 5 nm to 500 nm, more preferably 5 nm to 450 nm, more preferably 5 nm to 400 nm, more preferably 5 nm to 350 nm, more preferably 5 nm to 300 nm, more preferably 5 nm to 250 nm, more preferably 5 nm to 200 nm, more preferably 5 nm to 150 nm, more preferably 5 nm to 100 nm, more preferably 10 nm to 100 nm, more preferably 15 nm to 100 nm. Selecting the contour length of the scanning strands accordingly may allow the "range" of the scanning strands to be varied accordingly. However, the present invention is not limited to a particular length or contour length of the scanning strand.

Preferably, each scanning strand has a contour length which is greater than a contour length of each docking strand, preferably by at least 5%, more preferably by at least 10%, more preferably by at least 15%, more preferably by at least 20%, more preferably by at least 25%, more preferably by at least 30%, more preferably by at least 35%, more preferably by at least 40%, more preferably by at least 45%, more preferably by at least 50%. However, each scanning strand may have a contour length which is even greater relative to a contour length of each docking strand, e.g., by at least 100%, preferably by at least 200%, more preferably by at least 500%, more preferably by at least 1000%, more preferably by at least 1500%, more preferably by at least 2000%, more preferably by at least 2500%, more preferably by at least 3000%, more preferably by at least 3500%.

Preferably, a plurality of scanning strands are diffused, preferably freely diffused, in a solution which is applied to the target structure.

Preferably, a location of each marking is detected two-dimensionally and/or three-dimensionally along the target structure.

Preferably, at least one two-dimensional representation and/or at least one three-dimensional representation of the target structure is generated based on the detected locations of the markings.

The objection detailed at the beginning is also solved by a microscopy system for imaging at least one target structure, as defined by the features of independent claim 14. Preferred embodiments are defined by the features of the dependent claims, respectively.

The features, embodiments, and advantages, as discussed with respect to the method, apply to the microscopy system accordingly.

The microscopy system may include a plurality of docking strands. The docking strands may be applicable to the target structure and may be configured to dock to the target structure.

The microscopy system may further include at least one scanning strand which may be applicable to the target structure. Each scanning strand may include at least one marking which may be at least partially and at least temporarily attached to the respective scanning strand.

Each scanning strand may have a first docking section, which is configured to dock to the target structure, and at least a second docking section, which is configured to dock to at least one of the plurality of docking strands, at least partially simultaneously.

The marking may be detectable by at least one detection means to detect a location of the marking along the target structure, at least when the respective scanning strand is docked to a respective docking strand.

The microscopy system may further include one or more imaging strands which are applicable to the target structure, wherein the imaging strands each include at least one marking, respectively, which is at least partially attached to the respective imaging strand. At least one of the imaging strands may be configured to dock to an imaging strand docking section of at least one of the scanning strands.

The first docking section may be configured to provide a stronger bond than the second docking section and/or the first docking section maybe configured to provide a stronger bond than the imaging strand docking section and/or the imaging strand docking section may be configured to provide a stronger bond than the second docking section.

Preferably, the imaging strands are diffused, preferably freely diffused, in a solution which is applied to the target structure.

Preferably, the at least one second docking section is configured to sequentially dislodge from the respective docking strand and bond with one or more further docking strands.

Preferably, the second docking section is configured to sequentially dock to a plurality of docking strands, preferably at least three docking strands, more preferably at least four docking strands, more preferably at least five docking strands, more preferably at least six docking strands, while the first docking section is docked to the target structure at substantially the same location on the target structure.

Preferably, the marking is a fluorescent element, preferably a fluorogen, preferably a fluorogenic ligand, which is configured to emit a fluorescent signal which is detectable by the detection means to detect a location of the marking along the target structure, at least, preferably only, when the respective scanning strand is docked to a respective docking strand.

Preferably, the docking strands and/or the scanning strand(s) and/or the imaging strand(s) is/are configured as DNA strands having one or more DNA sequences or RNA strands having one or more RNA sequences.

Preferably, the first docking section has at least a first DNA/RNA sequence and the second docking section has at least a second DNA/RNA sequence which is different from the first DNA/RNA sequence.

Preferably, the first docking section and the second docking section are connected to each other by at least one link section.

Preferably, each scanning strand has a contour length of 5 nm to 700 nm, preferably 5 nm to 650 nm, more preferably 5 nm to 600 nm, more preferably 5 nm to 550 nm, more preferably 5 nm to 500 nm, more preferably 5 nm to 450 nm, more preferably 5 nm to 400 nm, more preferably 5 nm to 350 nm, more preferably 5 nm to 300 nm, more preferably 5 nm to 250 nm, more preferably 5 nm to 200 nm, more preferably 5 nm to 150 nm, more preferably 5 nm to 100 nm, more preferably 10 nm to 100 nm, more preferably 15 nm to 100 nm.

Preferably, each scanning strand has a contour length which is greater than a contour length of each docking strand, preferably by at least 5%, more preferably by at least 10%, more preferably by at least 15%, more preferably by at least 20%, more preferably by at least 25%, more preferably by at least 30%, more preferably by at least 35%, more preferably by at least 40%, more preferably by at least 45%, more preferably by at least 50%. However, each scanning strand may have a contour length which is even greater relative to a contour length of each docking strand, e.g., by at least 100%, preferably by at least 200%, more preferably by at least 500%, more preferably by at least 1000%, more preferably by at least 1500%, more preferably by at least 2000%, more preferably by at least 2500%, more preferably by at least 3000%, more preferably by at least 3500%.

Preferably, a plurality of scanning strands are diffused, preferably freely diffused, in a solution which is applicable to the target structure.

Preferably, the microscopy system is configured to two-dimensionally, and/or three-dimensionally detect a location of each marking along the target structure.

Preferably, the microscopy system is configured to generate at least one two-dimensional representation and/or at least one three-dimensional representation of the target structure based on the detected locations of the markings.

Preferably, the microscopy system further includes at least one imaging system configured to capture one or more images preferably a plurality of images, preferably sequentially, of at least a section of the target structure with a super resolution by means of at least one imaging system, at least after the docking strands and the scanning strands have been applied to the target structure; and/or the microscopy system is configured to generate one or more coordinates of at least some of the detected location(s) of the marking(s) along the target structure.

Preferably, the imaging system is configured to capture a plurality of images, preferably consecutive images, of at least a section of the target structure.

The objection detailed at the beginning is also solved by a set of strands for use with a microscope for imaging at least one target structure, as defined by the features of independent claim 15. Preferred embodiments are defined by the features of the dependent claims, respectively.

The features, embodiments, and advantages, as discussed with respect to the method, apply to the set accordingly.

The kit may include a plurality of docking strands which are applicable to the target structure and configured to dock to the target structure.

The kit may further include at least one scanning strand which is applicable to the target structure, wherein each scanning strand includes at least one marking which is at least partially and at least temporarily attached to the respective scanning strand.

Each scanning strand may have a first docking section, which is configured to dock to the target structure, and at least a second docking section, which is configured to dock to at least one of the plurality of docking strands, at least partially simultaneously,

The marking may be detectable by at least one detection means to detect a location of the marking along the target structure, at least when the respective scanning strand is docked to a respective docking strand.

Preferably, the set further includes one or more imaging strands which are applicable to the target structure, wherein the imaging strands each include at least one marking, respectively, which is at least partially attached to the respective imaging strand, wherein at least one of the imaging strands is configured to dock to an imaging strand docking section of at least one of the scanning strands.

Preferably, the first docking section is configured to provide a stronger bond than the second docking section; and/or the first docking section is configured to provide a stronger bond than the imaging strand docking section; and/or the imaging strand docking section is configured to provide a stronger bond than the second docking section.

Preferably, the at least one second docking section is configured to sequentially dislodge from the respective docking strand and bond with one or more further docking strands.

Preferably, the second docking section is configured to sequentially dock to a plurality of docking strands, preferably at least three docking strands, more preferably at least four docking strands, more preferably at least five docking strands, more preferably at least six docking strands, while the first docking section is docked to the target structure at substantially the same location on the target structure.

Preferably, the marking is a fluorescent element, preferably a fluorogen, preferably a fluorogenic ligand, which is configured to emit a fluorescent signal which is detectable by the detection means to detect a location of the marking along the target structure, at least, preferably only, when the respective scanning strand is docked to a respective docking strand.

Preferably, the docking strands and/or the scanning strand(s) and/or the imaging strand(s) is/are configured as DNA strands having one or more DNA sequences.

Preferably, the first docking section has at least a first DNA sequence and the second docking section has at least a second DNA sequence which is different from the first DNA sequence.

Preferably, the first docking section and the second docking section are connected to each other by at least one link section.

Preferably, each scanning strand has a contour length of 5 nm to 700 nm, preferably 5 nm to 650 nm, more preferably 5 nm to 600 nm, more preferably 5 nm to 550 nm, more preferably 5 nm to 500 nm, more preferably 5 nm to 450 nm, more preferably 5 nm to 400 nm, more preferably 5 nm to 350 nm, more preferably 5 nm to 300 nm, more preferably 5 nm to 250 nm, more preferably 5 nm to 200 nm, more preferably 5 nm to 150 nm, more preferably 5 nm to 100 nm, more preferably 10 nm to 100 nm, more preferably 15 nm to 100 nm.

Preferably, each scanning strand has a contour length which is greater than a contour length of each docking strand, preferably by at least 5%, more preferably by at least 10%, more preferably by at least 15%, more preferably by at least 20%, more preferably by at least 25%, more preferably by at least 30%, more preferably by at least 35%, more preferably by at least 40%, more preferably by at least 45%, more preferably by at least 50%.

Preferably, a plurality of scanning strands are diffused, preferably freely diffused, in a solution which is applicable to the target structure.

The following list of aspects provides preferred embodiments of the present disclosure:
1. A method for imaging at least one target structure, including:
   applying one or more docking strands to the target structure such that at least some of the docking strands dock to the target structure; and
   applying one or more scanning strands to the target structure, wherein at least some of the scanning strands have a first docking section, which docks to the target structure, and at least a second docking section, which docks to at least one of the plurality of docking strands, at least partially simultaneously,
   wherein at least some of the scanning strands include at least one marking which is at least partially and at least temporarily attached to the respective scanning strand and is detectable by at least one detection means to detect a location of the marking along the target structure, at least when the respective scanning strand is docked to a respective docking strand.
2. The method according to aspect 1, further including:
   applying one or more imaging strands to the target structure, wherein the imaging strands each include at least one marking, respectively, which is at least partially attached to the respective imaging strand, wherein at least one of the imaging strands docks to an imaging strand docking section of at least one of the scanning strands.
3. The method according to aspect 2, wherein the imaging strand docking section is arranged at an end section of the scanning strands, the end section being preferably substantially opposite from the first docking section.
4. The method according to aspect 2, wherein the second docking section is arranged between the imaging strand docking section and the first docking section.
5. The method according to aspect 1, further including:
   capturing one or more images, preferably a plurality of images, preferably sequentially, of at least a section of the target structure with a super resolution by means of at least one imaging system, at least after the docking strands and the scanning strands have been applied to the target structure; and/or
   generating one or more coordinates of at least some of the detected location(s) of the marking(s) along the target structure.
6. The method according to any of the preceding aspects, wherein:
   a stronger bond is generated at the first docking section than at the second docking section; and/or
   a stronger bond is generated at the first docking section than at the imaging strand docking section; and/or
   a stronger bond is generated at the imaging strand docking section than at the second docking section.
7. The method according to any of the preceding aspects, wherein:
   a time period, during which the first docking section is docked to the target structure, is longer than a time period, during which the second docking section is docked to the docking strand; and/or
   a time period, during which the first docking section is docked to the target structure, is longer than a time period, during which the imaging strand is docked to the imaging strand docking section; and/or
   a time period, during which the imaging strand is docked to the imaging strand docking section, is longer than a time period, during which the second docking section is docked to the docking strand.
8. The method according to any of the preceding aspects, wherein the at least one second docking section sequentially dislodges from the respective docking strand and bonds with one or more further docking strands.
9. The method according to any of the preceding aspects, wherein the scanning strands are configured to periodically dislodge from a first location on the target structure and dock to a different location on the target structure.
10. The method according to any of the preceding aspects, wherein the second docking section sequentially docks to a plurality of docking strands, preferably at least three docking strands, more preferably at least four docking strands, more preferably at least five docking strands, more preferably at least six docking strands, while the first docking section is docked to the target structure at substantially the same location on the target structure.
11. The method according to any of the preceding aspects, wherein the marking is a fluorescent element, preferably a fluorogen, preferably a fluorogenic ligand, which emits a fluorescent signal which is detectable by the detection means to detect a location of the marking along the target structure, at least, preferably only, when the respective scanning strand is docked to a respective docking strand.
12. The method according to any of the preceding aspects, wherein at least one marking is attached to an end section of the scanning strands, respectively, and/or at least one marking is attached to a section of the scanning strands which is arranged between the first docking section and the second docking section, respectively.
13. The method according to any of the preceding aspects, wherein the docking strands and/or the scanning strand(s) and/or the imaging strand(s) is/are configured as DNA strands having one or more DNA sequences.
14. The method according to aspect 13, wherein the first docking section has at least a first DNA sequence and the second docking section has at least a second DNA sequence which is different from the first DNA sequence.
15. The method according to any of the preceding aspects, wherein the first docking section and the second docking section are connected to each other by at least one link section.
16. The method according to any of the preceding aspects, wherein each scanning strand has a contour length of 5 nm to 700 nm, preferably 5 nm to 650 nm, more preferably 5 nm to 600 nm, more preferably 5 nm to 550 nm, more preferably 5 nm to 500 nm, more preferably 5 nm to 450 nm, more preferably 5 nm to 400 nm, more preferably 5 nm to 350 nm, more preferably 5 nm to 300 nm, more preferably 5 nm to 250 nm, more preferably 5 nm to 200 nm, more preferably 5 nm to 150 nm, more preferably 5 nm to 100 nm, more preferably 10 nm to 100 nm, more preferably 15 nm to 100 nm.
17. The method according to any of the preceding aspects, wherein each scanning strand has a contour length which is greater than a contour length of each docking strand, preferably by at least 5%, more preferably by at least 10%, more preferably by at least 15%, more preferably by at least 20%, more preferably by at least 25%, more preferably by at least 30%, more preferably by at least 35%, more preferably by at least 40%, more preferably by at least 45%, more preferably by at least 50%, more preferably by at least 100%, more preferably by at least 200%, more preferably by at least 500%, more preferably by at least 1000%, more preferably by at least 1500%, more preferably by at least 2000%, more preferably by at least 2500%, more preferably by at least 3000%, more preferably by at least 3500%.
18. The method according to any of the preceding aspects, wherein a plurality of scanning strands are diffused, preferably freely diffused, in a solution which is applied to the target structure.
19. The method according to any of the preceding aspects, wherein a location of each marking is detected two-dimensionally and/or three-dimensionally along the target structure.
20. The method according to any of the preceding aspects, wherein at least one two-dimensional representation and/or at least one three-dimensional representation of the target structure is generated based on the detected locations of the markings.
21. A microscopy system for imaging at least one target structure, including:
   a plurality of docking strands which are applicable to the target structure and configured to dock to the target structure; and
   at least one scanning strand which is applicable to the target structure, wherein each scanning strand includes at least one marking which is at least partially and at least temporarily attached to the respective scanning strand;
   wherein each scanning strand has a first docking section, which is configured to dock to the target structure, and at least a second docking section, which is configured to dock to at least one of the plurality of docking strands, at least partially simultaneously, and
   wherein the marking is detectable by at least one detection means to detect a location of the marking along the target structure, at least when the respective scanning strand is docked to a respective docking strand.
22. The microscopy system according to aspect 21, further including one or more imaging strands which are applicable to the target structure, wherein the imaging strands each include at least one marking, respectively, which is at least partially attached to the respective imaging strand, wherein at least one of the imaging strands is configured to dock to an imaging strand docking section of at least one of the scanning strands.
23. The microscopy system according to aspect 21 or 22, wherein:
   the first docking section is configured to provide a stronger bond than the second docking section; and/or
   the first docking section is configured to provide a stronger bond than the imaging strand docking section; and/or
   the imaging strand docking section is configured to provide a stronger bond than the second docking section.
24. The microscopy system according to any of aspects 21 to 23, wherein the at least one second docking section is configured to sequentially dislodge from the respective docking strand and bond with one or more further docking strands.
25. The microscopy system according to any of aspects 21 to 24, wherein the second docking section is configured to sequentially dock to a plurality of docking strands, preferably at least three docking strands, more preferably at least four docking strands, more preferably at least five docking strands, more preferably at least six docking strands, while the first docking section is docked to the target structure at substantially the same location on the target structure.
26. The microscopy system according to any of aspects 21 to 25, wherein the marking is a fluorescent element, preferably a fluorogen, preferably a fluorogenic ligand, which is configured to emit a fluorescent signal which is detectable by the detection means to detect a location of the marking along the target structure, at least, preferably only, when the respective scanning strand is docked to a respective docking strand.
27. The microscopy system according to any of aspects 21 to 26, wherein the docking strands and/or the scanning strand(s) and/or the imaging strand(s) is/are configured as DNA strands having one or more DNA sequences.
28. The microscopy system according to aspect 27, wherein the first docking section has at least a first DNA sequence and the second docking section has at least a second DNA sequence which is different from the first DNA sequence.
29. The microscopy system according to any of aspects 21 to 28, wherein the first docking section and the second docking section are connected to each other by at least one link section.
30. The microscopy system according to any of aspects 21 to 29, wherein each scanning strand has a contour length of 5 nm to 700 nm, preferably 5 nm to 650 nm, more preferably 5 nm to 600 nm, more preferably 5 nm to 550 nm, more preferably 5 nm to 500 nm, more preferably 5 nm to 450 nm, more preferably 5 nm to 400 nm, more preferably 5 nm to 350 nm, more preferably 5 nm to 300 nm, more preferably 5 nm to 250 nm, more preferably 5 nm to 200 nm, more preferably 5 nm to 150 nm, more preferably 5 nm to 100 nm, more preferably 10 nm to 100 nm, more preferably 15 nm to 100 nm.
31. The microscopy system according to any of aspects 21 to 30, wherein each scanning strand has a contour length which is greater than a contour length of each docking strand, preferably by at least 5%, more preferably by at least 10%, more preferably by at least 15%, more preferably by at least 20%, more preferably by at least 25%, more preferably by at least 30%, more preferably by at least 35%, more preferably by at least 40%, more preferably by at least 45%, more preferably by at least 50%, more preferably by at least 100%, more preferably by at least 200%, more preferably by at least 500%, more preferably by at least 1000%, more preferably by at least 1500%, more preferably by at least 2000%, more preferably by at least 2500%, more preferably by at least 3000%, more preferably by at least 3500%.
32. The microscopy system according to any of aspects 21 to 31, wherein a plurality of scanning strands are diffused, preferably freely diffused, in a solution which is applicable to the target structure.
33. The microscopy system according to any of aspects 21 to 32, wherein the microscopy system is configured to two-dimensionally, and/or three-dimensionally detect a location of each marking along the target structure.
34. The microscopy system according to any of aspects 21 to 33, wherein the microscopy system is configured to generate at least one two-dimensional representation and/or at least one three-dimensional representation of the target structure based on the detected locations of the markings.
35. The microscopy system according to any of aspects 21 to 34, wherein:
   the microscopy system further includes at least one imaging system configured to capture one or more images preferably a plurality of images, preferably sequentially, of at least a section of the target structure with a super resolution by means of at least one imaging system, at least after the docking strands and the scanning strands have been applied to the target structure; and/or
   the microscopy system is configured to generate one or more coordinates of at least some of the detected location(s) of the marking(s) along the target structure.
36. The microscopy system according to aspect 35, wherein the imaging system is configured to capture a plurality of images, preferably consecutive images, of at least a section of the target structure.
37. A set of strands for use with a microscope for imaging at least one target structure, including:
   a plurality of docking strands which are applicable to the target structure and configured to dock to the target structure; and
   at least one scanning strand which is applicable to the target structure, wherein each scanning strand includes at least one marking which is at least partially and at least temporarily attached to the respective scanning strand;
   wherein each scanning strand has a first docking section, which is configured to dock to the target structure, and at least a second docking section, which is configured to dock to at least one of the plurality of docking strands, at least partially simultaneously,
   wherein the marking is detectable by at least one detection means to detect a location of the marking along the target structure, at least when the respective scanning strand is docked to a respective docking strand.
38. The set according to aspect 37, further including one or more imaging strands which are applicable to the target structure, wherein the imaging strands each include at least one marking, respectively, which is at least partially attached to the respective imaging strand, wherein at least one of the imaging strands is configured to dock to an imaging strand docking section of at least one of the scanning strands.
39. The set according to aspect 37 or 38, wherein:
   the first docking section is configured to provide a stronger bond than the second docking section; and/or
   the first docking section is configured to provide a stronger bond than the imaging strand docking section; and/or
   the imaging strand docking section is configured to provide a stronger bond than the second docking section.
40. The set according to any of aspects 37 to 39, wherein the at least one second docking section is configured to sequentially dislodge from the respective docking strand and bond with one or more further docking strands.
41. The set according to any of aspects 37 to 40, wherein the second docking section is configured to sequentially dock to a plurality of docking strands, preferably at least three docking strands, more preferably at least four docking strands, more preferably at least five docking strands, more preferably at least six docking strands, while the first docking section is docked to the target structure at substantially the same location on the target structure.
42. The set according to any of aspects 37 to 41, wherein the marking is a fluorescent element, preferably a fluorogen, preferably a fluorogenic ligand, which is configured to emit a fluorescent signal which is detectable by the detection means to detect a location of the marking along the target structure, at least, preferably only, when the respective scanning strand is docked to a respective docking strand.
43. The set according to any of aspects 37 to 42, wherein the docking strands and/or the scanning strand(s) and/or the imaging strand(s) is/are configured as DNA strands having one or more DNA sequences.
44. The set according to any of aspects 37 to 43, wherein the first docking section has at least a first DNA sequence and the second docking section has at least a second DNA sequence which is different from the first DNA sequence.
45. The set according to any of aspects 37 to 44, wherein the first docking section and the second docking section are connected to each other by at least one link section.
46. The set according to any of aspects 37 to 45, wherein each scanning strand has a contour length of 5 nm to 700 nm, preferably 5 nm to 650 nm, more preferably 5 nm to 600 nm, more preferably 5 nm to 550 nm, more preferably 5 nm to 500 nm, more preferably 5 nm to 450 nm, more preferably 5 nm to 400 nm, more preferably 5 nm to 350 nm, more preferably 5 nm to 300 nm, more preferably 5 nm to 250 nm, more preferably 5 nm to 200 nm, more preferably 5 nm to 150 nm, more preferably 5 nm to 100 nm, more preferably 10 nm to 100 nm, more preferably 15 nm to 100 nm.
47. The set according to any of aspects 37 to 46, wherein each scanning strand has a contour length which is greater than a contour length of each docking strand, preferably by at least 5%, more preferably by at least 10%, more preferably by at least 15%, more preferably by at least 20%, more preferably by at least 25%, more preferably by at least 30%, more preferably by at least 35%, more preferably by at least 40%, more preferably by at least 45%, more preferably by at least 50%, more preferably by at least 100%, more preferably by at least 200%, more preferably by at least 500%, more preferably by at least 1000%, more preferably by at least 1500%, more preferably by at least 2000%, more preferably by at least 2500%, more preferably by at least 3000%, more preferably by at least 3500%.
48. The set according to any of aspects 37 to 47, wherein a plurality of scanning strands are diffused, preferably freely diffused, in a solution which is applicable to the target structure.

Preferred embodiments of the present invention are further elucidated below with reference to the figures. The described embodiments do not limit the present invention.
- Fig. 1: shows, in a schematic illustration, a method and a microscopy system according to an embodiment of the present disclosure in which a scanning strand is docked to a first docking strand;
- Fig. 2: shows, in a schematic illustration, the method and the microscopy system of Fig. 1 in which the scanning strand is docked to a second docking strand;
- Fig. 3: shows, in a schematic illustration, a method and a microscopy system according to a further embodiment of the present disclosure in which a scanning strand is docked to a first docking strand;
- Fig. 4: shows, in a schematic illustration, the method and the microscopy system of Fig. 3 in which the scanning strand is docked to a second docking strand;
- Fig. 5: shows measurement data of an exemplary measurement A;
- Fig. 6: shows measurement data of a further exemplary measurement B;
- Fig. 7: shows measurement data of a further exemplary measurement C.

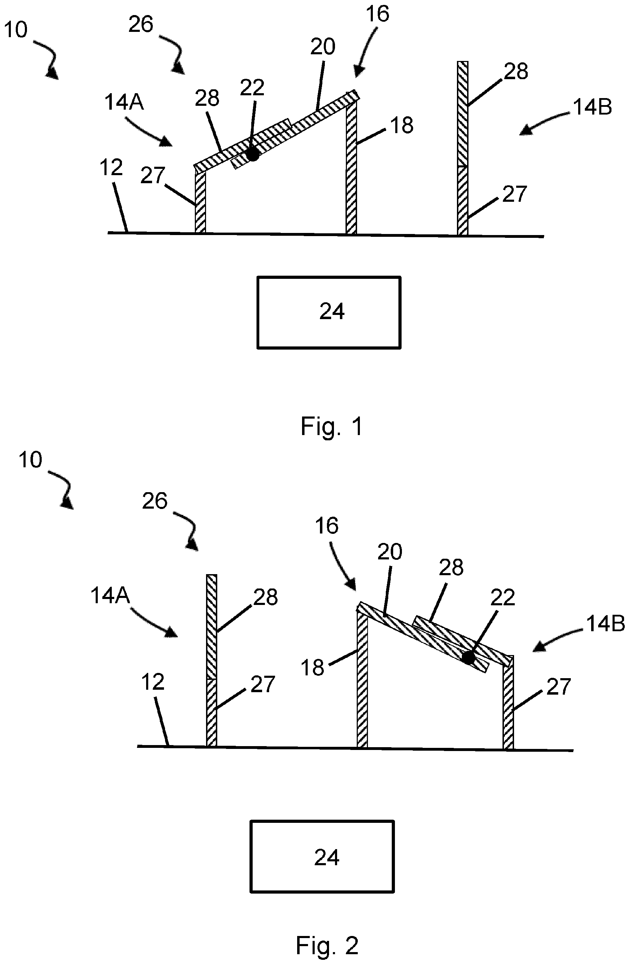

Figs. 1 and 2 show a method and a system 10 for imaging at least one target structure 12. The target structure 12 may be any type of structure which is to be imaged. The target structure 12 may include biological matter, preferably one or more cells, e.g., living cells, e.g., a single cell. For instance, the target structure 12 may be a strand, e.g., a DNA strand or an RNA strand, preferably an mRNA strand.

The system 10 may include a plurality of docking strands 14 which are applicable to the target structure 12 and configured to dock to the target structure 12 at different locations of the target structure 12.

The system 10 may further include at least one scanning strand 16, preferably a plurality of scanning strands 16, which is/are applicable to the target structure 12. The docking strands 14 and/or the scanning strand(s) 16 may be at least partially flexible, e.g., bendable.

The scanning strands 16 may have a plurality of subsections. In particular, each scanning strand 16 may have a first docking section 18, which is configured to dock to the target structure 12, and at least a second docking section 20, which is configured to dock to at least one of the plurality of docking strands 14, at least partially simultaneously.

The first docking section 18 may be configured to dock directly to the target structure 12. Alternatively, the first docking section 18 may be configured to dock indirectly, e.g., via an intermediate medium, e.g., an intermediate strand, to the target structure 12. For instance, the first docking section 18 may be configured to dock to at least one docking strand 14, as an intermediate structure between the first docking section 18 and the target structure 12. Thus, in this case, both the first docking section 18 and the second docking section 20 may dock to respective docking strands 14 at least partially simultaneously.

Each scanning strand 16 may further include at least one marking 22 which is at least partially and at least temporarily attached to the respective scanning strand 16.

The marking 22 may be detectable by at least one detection means 24 to detect a location of the marking 22 along the target structure 12, at least when the respective scanning strand 16 is docked to a respective docking strand 14. Preferably, a location of each marking 22 is detected two-dimensionally and/or three-dimensionally along the target structure 12.

The marking(s) 22 may be attached permanently to the respective scanning strand 16

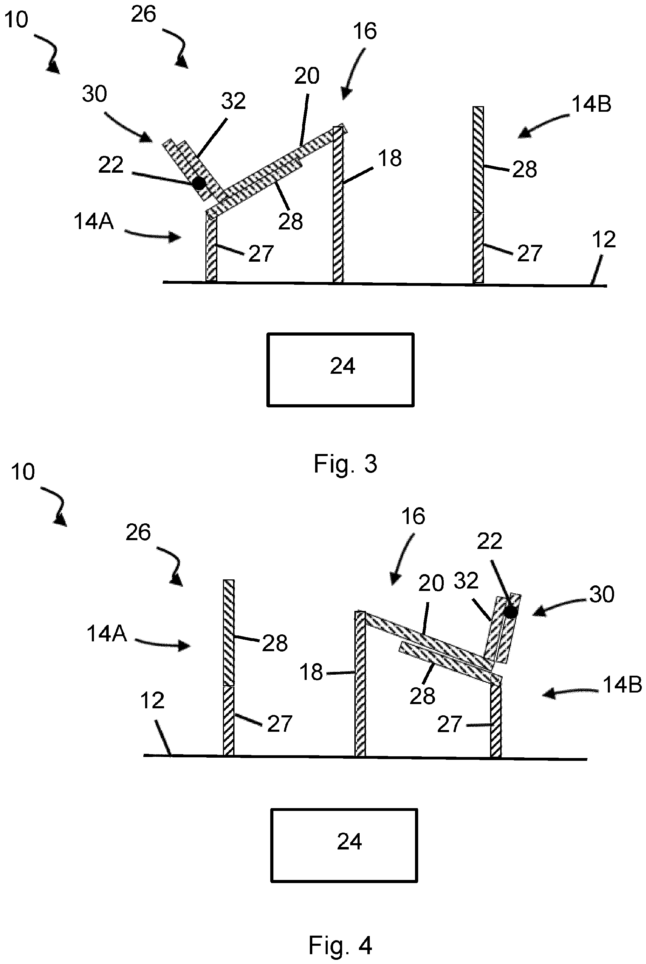

The marking(s) 22 may be attached directly to the respective scanning strand 16, preferably to the second docking section 20, as shown in Figs. 1 and 2. Alternatively, the marking(s) 22 may be attached indirectly to the respective scanning strand 16, e.g., via another strand, e.g., an imaging strand, as shown in Figs. 3 and 4 and described further below.

The scanning strand(s) 16 and the docking strands 14, and optionally further including one or more further types of strands, e.g., imaging strands as described further below, may also be referred to as a set 26 of strands for use with a microscope for imaging the target structure 12.

According to the method described herein, the docking strands 14 may be applied to the target structure 12 such that the docking strands 14 dock to the target structure 12. The one or more scanning strands 16 may be applied to the target structure 12. After having applied at least a portion of the docking strands 14, at least some of the docking strands 14 may dock to different locations on the target structure 12. Moreover, after having applied at least a portion of the scanning strand(s) 16 to the target structure 12, one or more of the scanning strand(s) 16 may dock to different locations on the target structure 12, via the first docking section 18 of the respective scanning strand 16. One or more of the scanning strand(s) 16 may dock to one of the docking strands 14A which is arranged within a range, i.e. within reach, of the scanning strand 16, as shown in Fig. 1.

The marking(s) 22 may be configured as a fluorescent element, preferably a fluorogen, preferably a fluorogenic ligand, which may be configured to emit a fluorescent signal which is detectable by the detection means 24 to detect a location of the marking 22 along the target structure 12, at least, preferably only, when the respective scanning strand 16 is docked to a respective docking strand 14.

The detection means 24 may include at least one imaging system. Thus, the marking(s) 22, more specifically the location of the marking(s) 22, may be detected by capturing one or more images, preferably a plurality of images, preferably sequentially, of at least a section of the target structure 12 with a super resolution by means of the imaging system. Additionally, or alternatively, the marking(s) 22, more specifically the location of the marking(s) 22, may be detected by generating one or more coordinates, and/or further data, e.g., one or more further pieces of information, suitable to indicate a location of the marking(s) 22, of at least some of the detected location(s) of the marking(s) 22 along the target structure 12.

The docking strands 14 may also include a plurality of different subsections, respectively. In particular, the docking strands 14 may include a first docking strand section 27 and at least one second docking strand section 28, respectively. For instance, the first docking strand section 27 may dock to the target structure 12, preferably permanently or at least for a duration which exceeds an imaging event of the detection means 24, and the second docking section 20 of the scanning strand 16 may be configured to transiently dock to the second docking strand section 28.

The second docking section 20 of the scanning strand(s) 16 may be configured to undock from the docking strand 14A and successively/sequentially dock to one or more further docking strands 14, e.g., docking strand 14B, which are arranged within a range of the scanning strand 16, as shown in Fig. 2, preferably, while the first docking section 18 of the scanning strand(s) 16 remains attached to substantially the same location on the target structure 12. In fact, the second docking section 20 may successively/sequentially dock to any number of docking strands 14, e.g., at least three docking strands 14, preferably at least four docking strands 14, more preferably at least five docking strands 14, more preferably at least six docking strands 14, while the first docking section 18 is docked to the target structure 12 at substantially the same location on the target structure 12.

This may allow the scanning strand 16 to "scan" an area about the location of the target structure 12 at which the first docking section 18 is docked by successively docking to a plurality of docking strands 14 and enabling respective locations of the marking 22, and thus of locations on the target structure 12, to be detected.

To facilitate transient docking and undocking of the second docking section 20 successively/sequentially to and from a plurality of docking strands 14, while the first docking section 18 is docked to the target structure 12 at substantially the same location on the target structure 12, a bonding strength and/or a bonding duration, i.e., a time period during which the respective docking section is docked to the respective target structure/docking strand, at the first docking section 18 may be configured to be greater than a bonding strength and/or a bonding duration at the second docking section 20.

For instance, the docking strands 14 and/or the scanning strand(s) 16 may be configured as DNA strands having one or more DNA sequences and/or RNA strands having one or more RNA sequences, wherein the first docking section 18 may have at least a first DNA/RNA sequence and the second docking section 20 may have at least a second DNA/RNA sequence which is different from the first DNA/RNA sequence. Configuring the first docking section 18 and the second docking section 20 to have different DNA/RNA sequences may allow the bonding strength and/or a bonding duration at the respective docking section to be varied. In this case, the first docking section 18 may be configured to dock directly to the target structure 12. Alternatively, the first docking section 18 may be configured to dock indirectly, e.g., via an intermediate medium, e.g., an intermediate strand, to the target structure 12. For instance, the first docking section 18 may be configured to dock to at least one docking strand 14, as an intermediate structure between the first docking section 18 and the target structure 12. Thus, in this case, both the first docking section 18 and the second docking section 20 may dock to respective docking strands 14 at least partially simultaneously via the different DNA/RNA sequences of the first docking section 18 and the second docking section 20.

The scanning strand(s) 16 may also be configured to periodically dislodge from a first location on the target structure 12 and dock to a different location on the target structure 12 to enable the scanning strand(s) 16 to "scan" different areas on the target structure 12, which may partially overlap.

Preferably, at least one two-dimensional representation and/or at least one three-dimensional representation of the target structure 12 is generated based on the detected locations of the markings 22.

Figs. 3 and 4 show, in schematic illustrations, a further embodiment of the method and the microscopy system 10. In particular, by contrast to the method and the microscopy system 10 shown in Figs. 1 and 2, the system 10 shown in Figs. 3 and 4 may further include one or more imaging strands 30 which may be applicable to the target structure 12. The marking(s) 22 may be at least partially attached to the imaging strands 30. At least one of the imaging strands 30 may be configured to dock to an imaging strand docking section 32 of at least one of the scanning strands 16.

Thus, in the configuration shown in Figs. 3 and 4, the marking(s) 22 may be attached indirectly to the respective scanning strand 16, i.e., via the imaging strands 30, rather than being attached directly to the respective scanning strand 16, as shown in Figs. 1 and 2 and described above. This may enable to more easily replace at least some of the markings 22, e.g., periodically, by new markings 22 by replacing at least some of the imaging strands 30, which have already been applied to the target structure 12, with replacement imaging strands 30, preferably with new markings 22 pre-attached to the replacement imaging strands 30. In particular, if the markings 22 are configured as a dye, a plurality of localizations at different docking strands 14 may cause a photon budget of the dye to be depleted, which may cause photobleaching, as discussed above. Hence, providing imaging strands 30 which can be replaced/regenerated may provide a relatively reliable and simple countermeasure to prevent, or at least reduce the risk of, photobleaching.

The imaging strands 30 may be configured as DNA strands having one or more DNA sequences or RNA strands having one or more RNA sequences.

According to the method described herein, the imaging strands 30 may be applied to the target structure 12. Once at least a portion of the imaging strands 30 has been applied to the target structure 12, one or more of the imaging strands 30 may dock to the imaging strand docking section 32 of at least one of the scanning strands 16.

The same marking 22 may be used for a plurality of successive docking and undocking events, in which the scanning strand 16 docks and undocks to and from a plurality of different docking strands 14. The duration/portion of the imaging process for which the same marking 22 is used may be determined/adjusted by configuring a bonding strength and/or a bonding duration, i.e., a time period during which the respective imaging strand 30 is docked to the respective imaging strand docking section 32 of the respective scanning strand 16, in particular relative to a bond strength and/or a bonding duration at the first docking section 18 and/or the second docking section 20. In particular, a stronger bond may be generated at the first docking section 18 than at the imaging strand docking section 32 and/or a stronger bond may be generated at the imaging strand docking section 32 than at the second docking section 20. In particular, a time period, during which the first docking section 18 is docked to the target structure 12, may be longer than a time period, during which the imaging strand 30 is docked to the imaging strand docking section 32 and/or a time period, during which the imaging strand 30 is docked to the imaging strand docking section 32, may be longer than a time period, during which the second docking section 20 is docked to the docking strand 14. The bonding strength and/or the bonding duration at the imaging strand docking section 32 may be selected to maximize the usage of the markings 22, while preventing, or at least reducing the risk of, photobleaching.

Thus, a hierarchy of bonding strengths and/or bonding durations may be achieved among the first docking section 18, the second docking section 20 and the imaging strand docking section 32. In particular, a ratio of the bonding strength and/or the bonding duration at the first docking section 18, the second docking section 20 and the imaging strand docking section 32 may be determined to increase the speed and/or the precision of the imaging process and/or maximize the usage of the markings 22, while preventing, or at least reducing the risk of, photobleaching.

As shown in Figs. 3 and 4, the imaging strand docking section 32 may be arranged at an end section of the scanning strands 16, the end section being preferably substantially opposite from the first docking section 18. Thus, the second docking section 20 may be arranged between the imaging strand docking section 32 and the first docking section 18. Alternatively, the imaging strand docking section 32 may be arranged between the first docking section 18 and the second docking section 20.

### Exemplary Measurements A to C

Exemplary measurements for the method and the system 10 are provided in the following.

### Buffer:

FOB refers to 1x TAE, 12 mM MgCl₂.

To suppress blinking and photobleaching, an oxidizing and reducing buffer system (1x TAE, 12 mM MgCl₂, 2 mM trolox/troloxquinone, 2.4 mM protocatechuic acid) was used in combination with an oxygen scavenging system. The 50x PCD buffer includes 2.8 mM PCD (protocatechuate 3,4-dioxygenase from pseudomonas sp.), 50% glycerol. 50 mM KCI, 100 mM Tris HCl and 1 mM EDTA-Na2·2H₂O. For measurements both buffers were mixed in a 1:50 ratio (50× PCD:Trolox/PCA), resulting in the imaging buffer.

### Graphene Coverslips

In order to prepare graphene coverslips, a wet-transfer approach was used to transfer the CVD-grown graphene onto glass coverslips. First, glass coverlips were cleaned with 1% Hellmanex and then subsequently washed twice in milliQ water, each step for 15 min in ultrasonication bath. Pieces of roughly 0.25 cm² were cut from PMMA/graphene/copper foil and let to float on 0.2 M ammonium persulfate for copper etching. After ^{~}3-4 h (when the copper foil was fully etched), PMMA/graphene was scooped gently with a clean coverslip and transferred to milliQ water to wash out the residues of ammonium persulfate. The washing step with the fresh milliQ water was repeated twice. Next, PMMA/graphene was scooped with a glass coverslip and carefully dried with a nitrogen stream. Samples were left for drying overnight. Next, ^{~}10 µl of PMMA (M_{w} = 15,000 g/mol) in chlorobenzene (50 mg/mL) was drop-casted to cure the protection layer of PMMA on graphene. After ^{~}30 min, when the solvent evaporated, the graphene-on-glass coverslip was placed in acetone for 7 min (×2) and in toluene for 7 min. After each step, the sample was dried with a nitrogen stream, and at last placed on active coal, heated on the heating plate to 230 C, for 30 min. Finally, the graphene-on-glass coverslip was removed from the active coal, and the incubation chamber (Grace BioLabs^{®}) was placed on a glass coverslip so that the graphene piece was in the middle of the chamber.

### Sample Preparation on Graphene

A DNA origami solution was immobilized on graphene-on-glass coverslips using the pyrene on the DNA origami structures for 2 min and then the sample was washed 3x using a buffer of 1x TAE and 12.5 mM MgCl₂. Next, gold nanorods for drift correction were immobilized on the surface via electrostatic interaction by incubating then gold nanorod for 2 min in a buffer of 1x TAE and 12.5 mM MgCl₂ and afterwards the sample was washed 3x with a buffer of 1x TAE and 12.5 mM MgCl₂.

### Measurement A:

### MINFLUX setup:

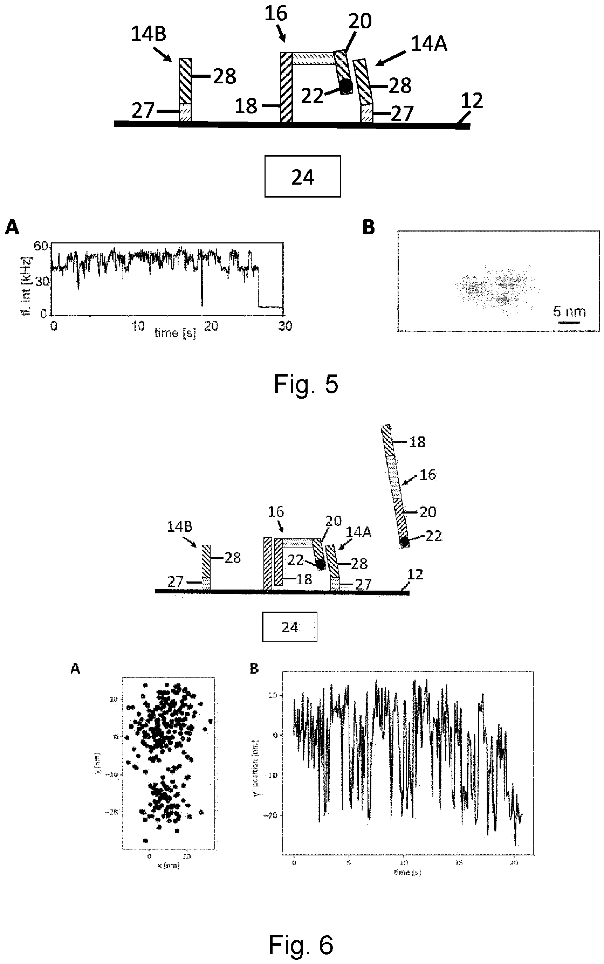

The MINFLUX data concerning measurement A, as shown in Fig. 5, was performed on a home built MINFLUX setup. The setup and the analysis are described in detail in the manuscript and the supporting information of the publication "pulsed interleaved MINFLUX", Nano Lett. 2021, 21, 1, 840-846 as Munich setup.

### Preparation of DNA Origami Structures

Two different DNA origami structures were used: rectangular DNA origami (based on Rothemund, Nature (2006) 440, 7082, 297-302 and Hübner et al., Nanoscale, 2022,14, 7898-7905 which is herewith incorporated by reference) and L-shaped DNA origami (Kaminska et al., Graphene Energy Transfer for Single-Molecule Biophysics, Biosensing, and Super-Resolution Microscopy. Adv. Mater. 2021, 33, 2101099 which is herewith incorporated by reference). The DNA origami structures were folded with 10-fold excess of oligonucleotide strands and a 100-fold excess of pyrene-modified oligonucleotides in comparison to the scaffold in 1× FOB buffer. Details of the folding program are found in Nickels et al. (Science 2016, 354(6310): 305-307 which is herewith incorporated by reference). After folding, 1× Blue Juice gel loading buffer was added to the folded DNA origami which was then purified via agarose-gel electrophoresis with 1.5% agarose gel in 50 mL of FOB buffer at 80 V for 1.5 h with 2 µL peqGREEN (ordered from VWR) per 100 µL buffer. The specific band for the nanostructure was extracted from the gel. Before putting the purified DNA origami solution onto graphene, the concentration was adjusted with FOB buffer to 75 pM.

Fig. 5A shows a fluorescent intensity trace of a 3D MINFLUX measurement of the marking of one exemplary scanning strand concerning measurement A.

Fig. 5B shows a 2D histogram along the x-z axis of the positions obtained from the detected marking 22.

In measurement A the L-shaped DNA origami is used. It is folded with 3 docking strands 14 and a scanning strand 16 protruding from the DNA origami. The positions of the protruding docking strands 14, e.g., 14A and 14B, are in a triangular pattern, with ^{~}6 nm spacing. In the middle of this triangular pattern, the scanning strand 16 protrudes from the origami and, such that it scans a distance of 3 nm. Here, both the scanning strand 16 and the docking strands 14 are permanently folded into the DNA origami. The docking strands 14 have a sequence of 23, 24 and 40 nucleotides respectively which are respectively complimentary to the scaffold strand.

The scanning strand 16 includes 4 parts. The first section 18 of 48 nucleotides is complementary to the scaffold of the DNA origami target 12 and binds to it. Then 12 nucleotides are used for flexibility of the scanning strand. The third part is the second docking section 20 with a 7 nucleotide sequence complimentary to the docking strands 14. The fourth part at the end is Cy3b as the fluorescent marking 22.

The three docking strands 14 of the DNA origami includes a 10 nucleotide sequence. Here, the first 3 nucleotides in section 27 from the origami enable flexibility of the docking strands 14 and 7 nucleotides in section 28 are complimentary to the second docking section 20 of the scanning strand 14.

For this measurement, first graphene is put on the coverslip and then the L-shaped origami is immobilized on graphene as described above. Then the imaging buffer is added and the chamber is sealed. The sample is imaged using the MINFLUX setup as an imaging system 24, described above. With an Avalanche Photon Diode, individual fluorescent traces are taken. In combination with MINFLUX and graphene, the 3D position of the fluorescent marking of the scanning strand 16 is tracked. Exemplary data is shown in Figure 5 resolving 3 positions with one marking.

### Sample Preparation on Glass:

The coverslips were first washed using ultrapure water, then cleaned using an ozone cleaner (PSD-UV4, Novascan Technologies, Inc., USA) and washed again using ultrapure water. For sample immobilization, the flow chamber was coated using biotin labeled bovine serum albumin (1 mg/mL Sigma-Aldrich Chemie GmbH) and StreptAvidin (1 mg/mL, Sigma-Aldrich Chemie GmbH). In the flow chamber, the DNA origami structures (100 pM in PBS and 750 mM NaCl) were immobilized due to the biotin-StrapAvidin binding.

### Widefield Setup and Data Analysis:

The measurements performed on a home-built wide-field microscope are based on an inverted Olympus IX71 microscope and use as an excitation source, a 532 nm/1 W laser (MPB Communications). The laser was spectrally cleaned-up by a filter (ZET532/10x, Chroma). For widefield illumination, the laser was focused in the back-focal plane of the objective (UPLXAPO 100X, numerical aperture (NA) = 1.45, working distance (WD) = 0.13, Olympus) in a illumination for total internal reflection fluorescence. Emission light was collected by the same objective and separated by dichroic beamsplitter (Dual Line zt532/ 640 rpc, AHF Analysentechnik). The emission light was spectrally filtered (ET 700/75, Chroma) before it was focused onto an EMCCD camera (iXon X3, DU-897, Andor). For data acquisition, the open source imageJ software Micro-Manager was used. The Using the Picasso software (J. Schnitzbauer et al., Super-Resolution Microscopy with DNA-PAINT Nature Protocols (2017). 12: 1198-1228), the data was then localized and rendered. Individual spots were then picked using Picasso and visualized using a custom python program.

### Measurement B:

Fig. 6A shows 2D positions of the marking 22 of one exemplary scanning strand concerning measurement B.

Fig. 6B shows a time trace of the x position of the marking of an exemplary scanning strand concerning measurement B.

For measurement B, a rectangular origami target 12 was folded with 2 docking strands 14 of ssDNA and an additional intermediate ssDNA strand for indirect docking of the scanning strand 16 with its first docking section 18 protruding from the origami. The intermediate strand has a 23 nucleotides sequence of ssDNA and acts as a docking site for the scanning strand. The first 5 nucleotides enable flexibility, while the other 18 nucleotides are complimentary to the first docking section 18 of the scanning strand 16. The docking strands 14 include a 10 nucleotide sequence. Here, the first 2 nucleotides in section 27 from the origami enable flexibility of the docking strand 14 and the next 8 nucleotides in section 28 are complimentary to the second docking section 20 of the scanning strand 16. The docking strands 14 are placed in a distance of 20 nm. The docking strands are folded into the DNA origami with a sequence complimentary to the DNA origami of 33 and 35 nucleotides respectively. The intermediate ssDNA strand for indirect docking of the scanning strand is placed in the middle between these docking strands and is permanently folded into the origami DNA with a sequence of 35 nucleotides.

The scanning strand 16 in this measurement includes 3 parts. The first section of 18 nucleotides is the docking section 18 complimentary to the intermediate ssDNA strands which dock the scanning strand 18 to the target 12. Then 3 nucleotides are used for flexibility of the scanning strand. The third part is the second docking section 20 with a 7 nucleotide sequence complimentary to the docking strands 14. Cy3B is covalently bound to the end of the second docking section as a fluorescent marking 22.

For this measurement, first the rectangular origami is immobilized on glass in a flow chamber, as described above. Next, the scanning strand is added in FOB buffer with a concentration of 10 nM at 30°C. Then the flow chamber is washed 3x with FOB and the imaging buffer is added. The flow chamber is then sealed and put on the widefield microscope as an imaging system 24. With an integration time of 500 ms, images are taken for 30 s. The resulting movie is then analyzed. Exemplary 2D data is shown in Fig. 6A and 6B, showing the scanning movement of the scanning strand 16 on two secondary docking sites.

### Measurement C:

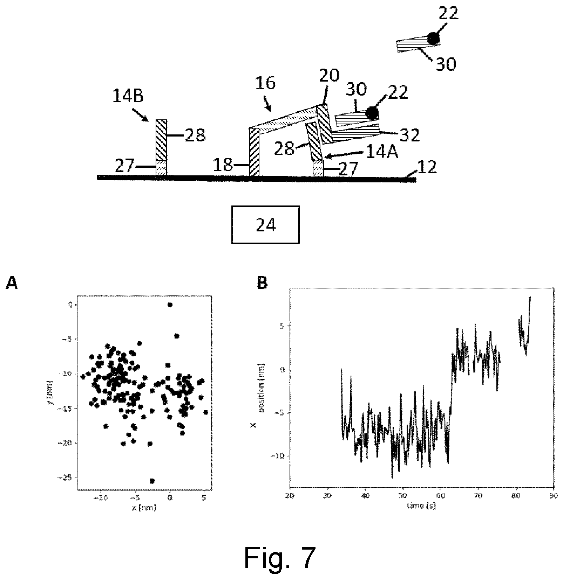

Fig. 7A shows 2D positions of the marking of one exemplary scanning strand concerning measurement C.

Fig. 7B shows a time trace of an x position of the marking of the scanning strand concerning measurement C.

In measurement C an L-shaped DNA origami is used. It is folded with 2 docking strands 14 and a scanning strand 16 protruding from the DNA origami. The docking strands are permanently folded into the DNA origami with a sequence of 48 nucleotides each. The positions of the three protruding strands are on a line, with 6 nm spacing. The middle strand is the scanning strand 16, such that it scans the distance of 12 nm between the docking strands 14.

The scanning strand 16 includes 5 parts. The first section 18 with 48 nucleotides is complimentary to the scaffold of the DNA origami and binds to it. Then 11 nucleotides are used for flexibility of the scanning strand. The third part is the second docking section 20 with an 8 nucleotide sequence complimentary to the docking strands 14. Then another 1 nucleotide is used for flexibility. Last is the imaging strand docking section 32 with 10 nucleotides.

The other two docking strands 14 of the DNA origami include a 10 nucleotide sequence. Here, the first 3 nucleotides in section 27 from the origami enable flexibility of the docking section and 8 nucleotides in section 28 are complimentary to the second docking section 20 of the scanning strand 16.

For this measurement, first the L-shaped origami is immobilized on glass in a flow chamber, as written above. Then the imaging buffer (here with a concentration of 75mM of MgCl₂) is added with 1nM of imaging strands 30 in the solution. The imaging strands 30 include a 10 nucleotide sequence complimentary to the imaging docking section 32 of the scanning strand 16 and an fluorescent marking 22 of Cy3B at the end. The flow chamber is then sealed and put on the widefield microscope as an imaging system 24. With an integration time of 200 ms, images are taken for 90 s. The resulting movie is then analyzed. Exemplary 2D data is shown in Fig. 7A and 7B, showing the scanning movement of the scanning strand 16 on two docking strands 14. The localization trace starts at 34 s, as an imager strand 30 with a marking 22 binds to the scanning strand 16 and has an intermittent gap as the imager strand unbinds and a new imager strand binds. In this gap the imaging strand 30 no imager is bound and 5 s later an imaging strand 30 again is transiently bound.

## Claims

1. A method for imaging at least one target structure (12), including:
applying a plurality of docking strands (14) to the target structure (12) such that the docking strands (14) dock to the target structure (12); and
applying one or more scanning strands (16) to the target structure (12), wherein each scanning strand (16) has a first docking section (18), which docks to the target structure (12), and at least a second docking section (20), which docks to at least one of the plurality of docking strands (14), at least partially simultaneously,
wherein each scanning strand (16) includes at least one marking (22) which is at least partially and at least temporarily attached to the respective scanning strand (16) and is detectable by at least one detection means (24) to detect a location of the marking (22) along the target structure (12), at least when the respective scanning strand (16) is docked to a respective docking strand (14).

2. The method according to claim 1, further including:
applying one or more imaging strands (30) to the target structure (12), wherein the imaging strands (30) each include at least one marking (22), respectively, which is at least partially attached to the respective imaging strand (30), wherein at least one of the imaging strands (30) docks to an imaging strand docking section (32) of at least one of the scanning strands (16).

3. The method according to claim 2, wherein the imaging strand docking section (32) is arranged at an end section of the scanning strands (16), the end section being preferably substantially opposite from the first docking section (18).

4. The method according to claim 1, further including:
capturing one or more images, preferably a plurality of images, preferably sequentially, of at least a section of the target structure (12) with a super resolution by means of at least one imaging system (24), at least after the docking strands (14) and the scanning strands (16) have been applied to the target structure (12); and/or
generating one or more coordinates of at least some of the detected location(s) of the marking(s) (22) along the target structure (12).

5. The method according to any of the preceding claims, wherein:
a stronger bond is generated at the first docking section (18) than at the second docking section (20); and/or
a stronger bond is generated at the first docking section (18) than at the imaging strand docking section (32); and/or
a stronger bond is generated at the imaging strand docking section (32) than at the second docking section (20).

6. The method according to any of the preceding claims, wherein:
a time period, during which the first docking section (18) is docked to the target structure (12), is longer than a time period, during which the second docking section (20) is docked to the docking strand (14); and/or
a time period, during which the first docking section (18) is docked to the target structure (12), is longer than a time period, during which the imaging strand (30) is docked to the imaging strand docking section (32); and/or
a time period, during which the imaging strand (30) is docked to the imaging strand docking section (32), is longer than a time period, during which the second docking section (20) is docked to the docking strand (14).

7. The method according to any of the preceding claims, wherein the at least one second docking section (20) sequentially dislodges from the respective docking strand (14) and bonds with one or more further docking strands (14).

8. The method according to any of the preceding claims, wherein the scanning strands (16) are configured to periodically dislodge from a first location on the target structure (12) and dock to a different location on the target structure (12).

9. The method according to any of the preceding claims, wherein the second docking section (20) sequentially docks to a plurality of docking strands (14), preferably at least three docking strands (14), more preferably at least four docking strands (14), more preferably at least five docking strands (14), more preferably at least six docking strands (14), while the first docking section (18) is docked to the target structure (12) at substantially the same location on the target structure (12).

10. The method according to any of the preceding claims, wherein the marking (22) is a fluorescent element, preferably a fluorogen, preferably a fluorogenic ligand, which emits a fluorescent signal which is detectable by the detection means (24) to detect a location of the marking (22) along the target structure (12), at least, preferably only, when the respective scanning strand (16) is docked to a respective docking strand (14).

11. The method according to any of the preceding claims, wherein at least one marking (22) is attached to an end section of the scanning strands (16), respectively, and/or at least one marking (22) is attached to a section of the scanning strands (16) which is arranged between the first docking section (18) and the second docking section (20), respectively.

12. The method according to any of the preceding claims, wherein the docking strands (14) and/or the scanning strand(s) (16) and/or the imaging strand(s) (30) is/are configured as DNA strands having one or more DNA sequences.

13. The method according to claim 13, wherein the first docking section (18) has at least a first DNA sequence and the second docking section (20) has at least a second DNA sequence which is different from the first DNA sequence.

14. A microscopy system (10) for imaging at least one target structure (12), including:
a plurality of docking strands (14) which are applicable to the target structure (12) and configured to dock to the target structure (12); and
at least one scanning strand (16) which is applicable to the target structure (12), wherein each scanning strand (16) includes at least one marking (22) which is at least partially and at least temporarily attached to the respective scanning strand (16);
wherein each scanning strand (16) has a first docking section (18), which is configured to dock to the target structure (12), and at least a second docking section (20), which is configured to dock to at least one of the plurality of docking strands (14), at least partially simultaneously, and
wherein the marking (22) is detectable by at least one detection means (24) to detect a location of the marking (22) along the target structure (12), at least when the respective scanning strand (16) is docked to a respective docking strand (14).

15. A set (26) of strands for use with a microscope for imaging at least one target structure (12), including:
a plurality of docking strands (14) which are applicable to the target structure (12) and configured to dock to the target structure (12); and
at least one scanning strand (16) which is applicable to the target structure (12), wherein each scanning strand (16) includes at least one marking (22) which is at least partially and at least temporarily attached to the respective scanning strand (16);
wherein each scanning strand (16) has a first docking section (18), which is configured to dock to the target structure (12), and at least a second docking section (20), which is configured to dock to at least one of the plurality of docking strands (14), at least partially simultaneously,
wherein the marking (22) is detectable by at least one detection means (24) to detect a location of the marking (22) along the target structure (12), at least when the respective scanning strand (16) is docked to a respective docking strand (14).

## Amended claims

### Amended claims in accordance with Rule 137(2) EPC.

1. A method for imaging at least one target structure (12), including:
applying a plurality of docking strands (14) to the target structure (12) such that the docking strands (14) dock to the target structure (12); and
applying one or more scanning strands (16) to the target structure (12), wherein each scanning strand (16) has a first docking section (18), which docks to the target structure (12), and at least a second docking section (20), which docks to at least one of the plurality of docking strands (14), at least partially simultaneously;
applying one or more imaging strands (30) to the target structure (12), wherein the imaging strands (30) each include at least one marking (22), respectively, which is at least partially attached to the respective imaging strand (30),
wherein at least one of the imaging strands (30) docks to an imaging strand docking section (32) of at least one of the scanning strands (16),
wherein the at least one marking (22) is detectable by at least one detection means (24) to detect a location of the marking (22) along the target structure (12), at least when the respective scanning strand (16) is docked to a respective docking strand (14), and
wherein a stronger bond is generated at the imaging strand docking section (32) than at the second docking section (20).

2. The method according to claim 1, wherein the imaging strand docking section (32) is arranged at an end section of the scanning strands (16), the end section being preferably substantially opposite from the first docking section (18).

3. The method according to claim 1, further including:
capturing one or more images, preferably a plurality of images, preferably sequentially, of at least a section of the target structure (12) with a super resolution by means of at least one imaging system (24), at least after the docking strands (14) and the scanning strands (16) have been applied to the target structure (12); and/or
generating one or more coordinates of at least some of the detected location(s) of the marking(s) (22) along the target structure (12).

4. The method according to any of the preceding claims, wherein:
a stronger bond is generated at the first docking section (18) than at the second docking section (20); and/or
a stronger bond is generated at the first docking section (18) than at the imaging strand docking section (32).

5. The method according to any of the preceding claims, wherein:
a time period, during which the first docking section (18) is docked to the target structure (12), is longer than a time period, during which the second docking section (20) is docked to the docking strand (14); and/or
a time period, during which the first docking section (18) is docked to the target structure (12), is longer than a time period, during which the imaging strand (30) is docked to the imaging strand docking section (32); and/or
a time period, during which the imaging strand (30) is docked to the imaging strand docking section (32), is longer than a time period, during which the second docking section (20) is docked to the docking strand (14).

6. The method according to any of the preceding claims, wherein the at least one second docking section (20) sequentially dislodges from the respective docking strand (14) and bonds with one or more further docking strands (14).

7. The method according to any of the preceding claims, wherein the scanning strands (16) are configured to periodically dislodge from a first location on the target structure (12) and dock to a different location on the target structure (12).

8. The method according to any of the preceding claims, wherein the second docking section (20) sequentially docks to a plurality of docking strands (14), preferably at least three docking strands (14), more preferably at least four docking strands (14), more preferably at least five docking strands (14), more preferably at least six docking strands (14), while the first docking section (18) is docked to the target structure (12) at substantially the same location on the target structure (12).

9. The method according to any of the preceding claims, wherein the marking (22) is a fluorescent element, preferably a fluorogen, preferably a fluorogenic ligand, which emits a fluorescent signal which is detectable by the detection means (24) to detect a location of the marking (22) along the target structure (12), at least, preferably only, when the respective scanning strand (16) is docked to a respective docking strand (14).

10. The method according to any of the preceding claims, wherein at least one marking (22) is attached to an end section of the scanning strands (16), respectively, and/or at least one marking (22) is attached to a section of the scanning strands (16) which is arranged between the first docking section (18) and the second docking section (20), respectively.

11. The method according to any of the preceding claims, wherein the docking strands (14) and/or the scanning strand(s) (16) and/or the imaging strand(s) (30) is/are configured as DNA strands having one or more DNA sequences.

12. The method according to claim 11, wherein the first docking section (18) has at least a first DNA sequence and the second docking section (20) has at least a second DNA sequence which is different from the first DNA sequence.

13. A microscopy system (10) for imaging at least one target structure (12), including:
a plurality of docking strands (14) which are applicable to the target structure (12) and configured to dock to the target structure (12);
at least one scanning strand (16) which is applicable to the target structure (12); and
one or more imaging strands (30) which are applicable to the target structure (12), wherein the imaging strands (30) each include at least one marking (22), respectively, which is at least partially attached to the respective imaging strand (30), wherein at least one of the imaging strands (30) is configured to dock to an imaging strand docking section (32) of at least one of the scanning strands (16);
wherein each scanning strand (16) has a first docking section (18), which is configured to dock to the target structure (12), and at least a second docking section (20), which is configured to dock to at least one of the plurality of docking strands (14), at least partially simultaneously,
wherein the marking (22) is detectable by at least one detection means (24) to detect a location of the marking (22) along the target structure (12), at least when the respective scanning strand (16) is docked to a respective docking strand (14), and
wherein the imaging strand docking section (32) is configured to provide a stronger bond than the second docking section (20).

14. A set (26) of strands for use with a microscope for imaging at least one target structure (12), including:
a plurality of docking strands (14) which are applicable to the target structure (12) and configured to dock to the target structure (12); and
at least one scanning strand (16) which is applicable to the target structure (12);
one or more imaging strands (30) which are applicable to the target structure (12), wherein the imaging strands (30) each include at least one marking (22), respectively, which is at least partially attached to the respective imaging strand (30), wherein at least one of the imaging strands (30) is configured to dock to an imaging strand docking section (32) of at least one of the scanning strands (16);
wherein each scanning strand (16) has a first docking section (18), which is configured to dock to the target structure (12), and at least a second docking section (20), which is configured to dock to at least one of the plurality of docking strands (14), at least partially simultaneously,
wherein the marking (22) is detectable by at least one detection means (24) to detect a location of the marking (22) along the target structure (12), at least when the respective scanning strand (16) is docked to a respective docking strand (14), and
wherein the imaging strand docking section (32) is configured to provide a stronger bond than the second docking section (20).
